(19) Europäisches Patentamt
European Patent Office
Office européen des brevets

(11) Publication number: 0 162 464 B1

# (12) EUROPEAN PATENT SPECIFICATION

(45) Date of publication of patent specification: 15.03.89

(21) Application number: 85106312.3

(22) Date of filing: 22.05.85

(51) Int. Cl.⁴: C 07 D 295/14, C 07 C 121/47, C 07 C 121/34, C 07 C 120/00, C 07 D 213/61, C 07 D 213/64, C 07 D 213/09

(54) **Synthesis of 2-substituted-5-methylpyridines from methylcyclo-butanecarbonitrile, valeronitrile and pentenonitrile intermediates.**

(30) Priority: 23.05.84 US 613216

(43) Date of publication of application: 27.11.85 Bulletin 85/48

(45) Publication of the grant of the patent: 15.03.89 Bulletin 89/11

(84) Designated Contracting States: BE DE FR GB IT

(56) References cited:
EP-A-0 046 735
EP-A-0 108 483
US-A-3 481 936
US-A-4 245 098

TETRAHEDRON, vol. 24, April 1968, MADSEN et al. "Enamine chemistry-X. The reaction of enamines with alpha-chloroacrylonitrile", pp. 3369-3379

(73) Proprietor: ICI AMERICAS INC
Concord Pike & New Murphy Road
Wilmington Delaware 19897 (US)

(72) Inventor: Hartmann, Ludwig Albert
211 Nassau Drive
Wilmington Delaware (US)
Inventor: Stephen, John Fergus
2000 William Penn Boulevard
West Chester Pennsylvania (US)

(74) Representative: Baillie, Iain Cameron et al
c/o Ladas & Parry Isartorplatz 5
D-8000 München 2 (DE)

(56) References cited:
JOURNAL OF THE CHEMICAL SOCIETY, 1964, Part II, London, FLEMING et al. "The reaction of enamines with electrophilic olefins. A synthesis of cyclobutanes", pp. 2156-2174

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 162 464 B1

**Description**

The present invention is directed to a new method of synthesis of 2-substituted-5-methylpyridines and to novel intermediates produced in the synthesis. In general, the process is directed to reacting propionaldehyde, a secondary amine, and a haloacrylonitrile in a stepwise Michael-type addition to form an amino substituted methylcyclobutanecarbonitrile intermediate which is thereafter subjected to acid hydrolysis to form valeronitrile or pentenonitrile derivatives which are thereafter cyclized to form 2-halo or 2-hydroxy-5-methylpyridines.

Various 4-(5-halomethyl-2-pyridyloxy)phenoxy compounds are known to be useful as herbicides as disclosed in European Published Patent Application No. 0 000 483, United Kingdom Patent Specifications 1,599,121 and 1,599,126 and U.S. Patents 4,184,041 and 4,317,913. For example, butyl 2-[4-(5-trifluoromethyl-2-pyridyloxy)-phenoxy] propionate which is also known as fluazifop-butyl is an effective grass herbicide which can be used in fields where broad leaved crops such as cotton and soybeans are cultivated. Important starting materials for such pyridyloxyphenoxy compounds are the 2-halo-5-trichloromethylpyridines such as 2-chloro-5-trichloromethylpyridine described in U.S. Patant 4,317,913. Such 2-halo-5-trichloromethylpyridines in turn may be prepared by chlorinating under ultra-violet light irradiation a 2-halo-5-methylpyridine as described in U.S. Patent 4,152,328.

An object of the present invention is to provide an efficient economical and reliable synthesis of 2-substituted-5-methylpyridines as well as for certain intermediates useful in their synthesis. It is another object to provide novel intermediates for use in manufacturing the 2-substituted-5-methylpyridines such as 2-halo-4-formylvaleronitrile, and 4-formyl-2-penteno-nitrile.

A further object of the present invention is to provide for a method for preparing 2-halo-5-methylpyridines without utilizing pyridine or 3-picoline starting materials thus avoiding the problems of byproduct formation in the halogenation reaction.

Still another object is to provide an alternate process to that presented in EP—A2—0108483.

The present invention comprises a method for the synthesis of a 5-methylpyridine derivative of the formula (I):

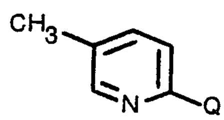

(I)

wherein Q is OH or X, and X is chloro or bromo by cyclization in the presence of an acid catalyst of novel compounds having the formula (II) or formula (III):

$$OCHCH(CH_3)CH_2CH(X)CN \qquad (II)$$

$$OCHCH(CH_3)CH{=}CHCN \qquad (III)$$

which are made by acidic hydrolysis under mild conditions (formula II) or stronger conditions (formula III) of a novel cyclobutane derivative having a general formula (IV):

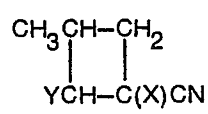

(IV)

wherein Y is —$NR^1R^2$ wherein $R^1$ and $R^2$ are selected from individual substituted and unsubstituted alkyl groups having 1—6 carbon atoms and groups connected to form 5- or 6-membered heterocyclic rings and wherein X is chlorine or bromine.

Preparation of Methylcyclobutane Carbonitrile Derivatives

Compounds of the general formula (IV) are made by condensing propionaldehyde in a Michael-type 2-step addition with an acrylic compound of the following formula (V):

$$CH_2{=}C(X)CN \qquad (V)$$

The Michael addition may be conducted as known in the art such as at a temperature of about 0 to 100°C neat or in the presence of an inert solvent and optionally in the presence of a base catalyst. The compound of formula (IV) may be recovered by extraction, chromatography or distillation. Preferably the Michael addition is carried out in two steps by first reacting the propionaldehyde with a secondary amine of the formula $HNR^1R^2$ wherein $R^1$ and $R^2$ are independently organic moieties which may be attached to each other to form a ring, to form directly or through an intermediate aminal of formula (VI) an enamine having structure (VII):

$CH_3—CH_2—CHYY$ (VI) or $CH_3—CH{=}CHY$ (VII)

and mixtures thereof wherein Y is $—NR^1R^2$. In particular $R^1$ and $R^2$ include individual substituted and unsubstituted alkyl of about 1—6 carbon atoms such as methyl, ethyl, propyl and butyl and pentyl and, $R^1$ and $R^2$ are connected substituted and unsubstituted heterocyclic rings such as 5- or 6-membered heterocyclic rings for example to define the secondary amine such as pyrrolidine, piperidine and morpholine. This reaction may be conducted at about −10 to 35°C preferably in the presence of an alkali or alkali earth metal carbonate, sulfate halide or oxide for example calcium sulfate, magnesium sulfate, calcium chloride, sodium sulfate, magnesium oxide, potassium carbonate, calcium oxide or even molecular sieves as disclosed by D. Roelofsen et al. in Recueil, Vol. 91, pages 605—610 (1972) with at least 2 moles of secondary amine per mol of propionaldehyde. The secondary amine must be used in excess in view of the formation of an aminal of the formula $CH_3CH_2CH(NR^1R^2)_2$ which is formed in addition to the enamine of the formula $CH_3CH{=}CH(NR^1R^2)$. The aminal and enamine mixture in mol ratios of 1:1 and 1:6 usually occurs as a liquid which may be heated to distill unreacted secondary amine and thereafter combined with a solvent such as acetonitrile and reacted with α-halocrylonitrile under mild conditions to form the cyclobutane derivative of formula (IV). The synthesis of cyclobutanes of this type is described in detail by I. Fleming et al. in the Journal of the Chemical Society, pages 2165—2174 (1964) and U.S. Patents 3,051,622; 3,133,924; 3,369,024; 3,481,936 and 3,481,939. The 1-chloro-2-(4-morpholino)-3-ethyl-cyclobutane carbonitrile homolog has been made by Madsen and Lawessen, Tetrahedron, *24*, 3369 (1968) by the addition of α-chloroacrylonitrile to morpholinobutene in acetonitrile solvent. While the reaction may be carried out neat it is preferred to carry it out in the presence of a solvent such as an ether, ester, halogenated alkane, ketone or nitrile solvent for example acetonitrile. The reaction may be carried out at room temperature up to the boiling point of the haloacrylic compound for example up to about 120°C with the higher temperature of this range being advantageously used to complete the reaction. Satisfactory yields are obtained at temperatures of 25°—80°C. In carrying out the reaction it is preferred that the aminal/enamine mixture be cooled to −5 to 20°C with dropwise addition of the haloacrylonitrile followed by warming to the range of room temperature to about the boiling of the halonitrile solvent.

The α-haloacrylonitrile of formula (V) are known and made by halogenation of acrylonitrile to form the 2,3-dihalopropionitrile followed by dehydrohalogenation.

Preparation of 2-Halo-4-formylvaleronitrile

The compounds of formula (II) are made by hydrolyzing to cleave cyclobutanes of formula (IV) with recovery of the secondary amine $HNR^1R^2$. The reaction may be conducted in an aqueous acidic medium such as in the presence of an aqueous acid such as acetic, sulfuric, hydrochloric, phosphoric or p-toluene sulfonic acid optionally in a solvent such as nitrile, ether, ester, halogenated alkane or ketone. A solvent system is preferred which allows the hydrolysis product to separate as a water insoluble layer during the reaction. The hydrolysis is carried out under controlled conditions at a temperature of about 25° to 80°C at a pH of about 1.5 to 4.5. A solvent other than the aqueous acidic reaction medium need not be present. Aldehydes of formula (II) may be recovered by phase separation and extraction of the aqueous acid solution containing the secondary amine with a neutral organic solvent such as ethyl acetate or methylene chloride.

Preparation of 4-Formyl-2-pentenonitrile

In the preparation of the material of formula (II) as described above a minor amount of a dehydrohalogenated product is formed as formula (III). However, if the material of formula (IV) is heated at 80—105°C especially in the presence of aqueous organic acid the product of formula (III) is obtained directly.

Preparation of 2-Halo-5-methylpyridine

The aldehydes of formula (II) or (III) are converted directly to the pyridine of formula (I) (when X=Cl) by acid catalyzed ring closure. For example, the compound of formula (II) may be cyclized with hydrogen halide such as HCl, and sulfuric acid, phosphoric acid or sulfonic acid at a temperature of 25 to about 100°C neat or in a solvent such as halogenated hydrocarbon or dimethyl formamide. Similar ring closures and reaction conditions are described in U.S. Patent 4,245,098 and EP—A—0046735 (March 3, 1982). The preparation of 2,3,5-trichloropyridine from 2,4,4-trichloro-4-formylbutyronitrile is disclosed in these references.

In the case with the material of formula (III) the acid catalyzed ring closure is carried out with either hydrogen bromide or hydrogen chloride depending upon the halide intended.

Preparation of 2-Hydroxy-5-methylpyridine

When the compounds of formula (II) and (III) are heated in the presence of p-toluene sulfonic acid or other strong organic acids or mineral acids at temperatures of 110—135°C, 2-hydroxy-5-methylpyridine is formed in high yields.

N. P. Susherina et al. in Chemical Abstracts, Vol. 55, page 7410e, and A. I. Meyers in J. Organic Chemistry, Vol. 29, page 1435—1438 (1964) and U.S. Patent 3,944,559 disclose ring closures of α-

ketonitriles and of 2-(2-cyanoalkyl)cyclohexanones and the preparation of certain 3,4-dihydro-2-pyridones and 2-pyridones.

In the following examples and throughout the specification the following abbreviations are used: °C (degrees centigrade); ml (milliliters); g (grams); m (mols); mm (millimeters); GLC (gas liquid chromatography); GC/MS (gas chromatograph-mass spectrometry); IR (infra red); NMR (nuclear magnetic resonance); mp (melting point); bp (boiling point); DSC (differential scanning calorimetry); MS (mass spectrometry); and the conventional symbols for the chemical elements. All proportions expressed herein are parts by weight unless otherwise specified.

Preparation A

Preparation of 1-(N-morpholino)prop-1-ene (MP) and 1,1-bis(N-morpholino)propane(aminal)

A slurry was prepared from 200 g distilled morpholine and 4.25 g anhydrous potassium carbonate. This slurry was cooled to 25°C and stirred while propionaldehyde (61.9 g) was added dropwise. The temperature was held at 25°C by cooling with an ice-bath during the addition (40 minutes). Reaction was continued at 25—30°C for 2 hours prior to vacuum stripping at 30—50°C under moderate vacuum (20—40 mm Hg). The product (aminal) was filtered and was then subjected to thermal cracking under vacuum, using an efficient fractionating column to avoid losses of aminal or morpholino-propene. Cracking to morpholinopropene was carried out for about 2 hours at a pot temperature of 65—85°C with the reflux adjusted so that a still-head temperature of about 50°C/40 mm Hg could be maintained. The product was a light-colored liquid of low viscosity, weighing 89.1 g. The mol ratio of MP to aminal, as determined by NMR analysis, was 2.5:1 (this corresponds to about 60% MP/40% aminal in weight percent).

Preparation B

Preparation of 1-(N-morpholino)prop-1-ene (MP) and 1,1-bis(morpholino)propane(aminal)

A slurry of 200 g morpholine and 0.85 g anhydrous potassium carbonate was treated with 61.9 g propionaldehyde as in Example 1. Partial cracking to MP was carried out for about 1 hour at 80°C pot temperature and the product composition then corresponded to about 2:1 MP:aminal. One half of the product (63.6 g) was cracked further for one hour at 82—89°C pot temperature and 40 mm Hg vacuum, with the still-head temperature at 45—51°C. The yield of product was 40.8 g and the composition (mol ratio) by NMR analysis was 5:1 MP:aminal (weight percent: 75% MP/25% aminal).

Example 1

Preparation of 1-Chloro-2-(4-morpholino)-3-methylcyclobutane carbonitrile

A 50.6 g portion of the material made in Preparation A was diluted with 50 ml acetonitrile and treated gradually with a solution of 41.2 g of α-chloroacrylonitrile in 40 ml of acetonitrile over a period of 1.5 hours at 25°C. The product was heated at 50°C for 3 hours and was then stripped at 50°C under moderate low pressure (150—40 mm Hg). The product residue weighed 90.4 g. It was chilled at 0°C and crystallized. A slurry with one part hexane was filtered to obtain 33.2 g pure product (mp 77—78°C). Analysis: C, 55.57; H, 7.09; N, 12.95; Cl, 16.34%. Calculated: C, 55.95; H, 7.04; N, 13.04; Cl, 16.51%. The product was purified by recrystallization from a mixture of toluene and hexane (1 part sample, 1 part toluene, 2 parts hexane (mp 84—85°C). NMR analysis showed that the cyclobutane product was obtained.

Example 2

Preparation of 1-Bromo-2-(4-morpholino)-3-methylcyclobutane carbonitrile

A 50 g portion of the MP-aminal product of Preparation B, is diluted with 50 ml acetonitrile and treated gradually with a solution of 60 g (α-bromoacrylonitrile in 60 ml acetonitrile at 25°C (1.5 hours). The product is heated at 50°C for 3 hours and is then vacuum stripped at 50°C under moderate vacuum (expected yield = 110 g). The product may be crystallized upon cooling and recovered as a solid after filtration. It may be recrystallized from hexane/acetonitrile.

Example 3

Preparation of 2-Chloro-4-formylvaleronitrile

12 g of the product of Example 1 having mp of 77—78°C and 7.2 ml acetonitrile was stirred and treated with 7.2 ml 8N $H_2SO_4$ at room temperature in portions until all was dissolved. This reaction mixture was then heated to 60°C and held at that temperature for 2 hours. The product mixture was separated in a separatory funnel. The upper product layer (12.0 g) was purified by adding 25 ml toluene and washing twice with 8 ml water. The solvent was then vacuum stripped at 55°C/30 mm Hg and 6.7 g of product was obtained as residue. The content of the valeronitrile derivative was 94% as determined by GLC. Analysis: C, 48.45; H, 5.65; Cl, 22.40; N, 10.52%. Calculated: C, 49.5; H, 5.53; Cl, 24.35; N, 9.62%. The structure was confirmed by GC/MS. The product was further purified by distillation at vapor temperature 92—95°C/ 4 mm Hg and was obtained as a colorless non-viscous liquid.

Example 4

Preparation of 2-Bromo-4-formylvaleronitrile

A mixture of 50 g cyclobutane carbonitrile product of Example 2 and 30 ml acetonitrile is treated at

room temperature with 25 ml 8N $H_2SO_4$ in portions until all is dissolved. The hydrolysis is then completed at 60°C (2 hours). The upper layer (product) is separated and purified by adding 75 ml toluene and washing several times with 20 ml water. The solvent is then vacuum-stripped at 55°C/30 mm Hg and 2-bromo-4-formylvaleronitrile is obtained as a liquid residue.

Example 5

Synthesis of 2-Chloro-4-formylvaleronitrile

A sample of 40.1 g non-crystalline cyclobutanecarbonitrile derivative, obtained as hexane-insoluble product (lower layer) after crystallization and filtration (Example 1), was treated with 24 ml 8N $H_2SO_4$, dropwise at 28—34°C while stirring and cooling. The reaction mixture was heated at 62—64°C for 1 hour and the 2-phase reaction product was then separated. 2-Chloro-4-formylvaleronitrile was isolated as the upper layer. The yield was 14.7 g; GLC analysis showed that the product was predominantly 2-chloro-4-formylvaleronitrile with a small amount of 4-formyl-2-pentenonitrile also present.

Example 6

Synthesis of 4-Formyl-2-pentenonitrile

A sample of 5 g crystallized cyclobutanecarbonitrile product (Example 1) was mixed with 10 ml glacial acetic acid and 10 ml water. The reaction mixture was stirred and heated at 80°C for 20 minutes and was then vacuum stripped at 100°C/20 mm Hg after a period of 16 hours at room temperature. The product residue was diluted with 18 g toluene and residual aqueous phase was separated. The toluene solution of product was washed two times with 5 ml $H_2O$ and vacuum stripped at 50°C/20 mm Hg. The yield of product was 1.1 g. The product was predominantly 4-formyl-2-pentenonitrile by GLC (N, 12.6%; calc.: N, 12.8%; residual Cl, 3%).

Example 7

Preparation of 2-Chloro-5-methylpyridine

A 2 g sample of undistilled product from Example 3 was dissolved in 8 g dimethyl formamide. This solution was stirred while HCl gas was introduced very slowly as the temperature was raised to 60°C. The temperature was held at 60—85°C for 1 hour and then at 100°C for 7 hours while HCl was introduced slowly throughout. An additional 3 g of dimethyl formamide was added after 3 hours at 100°C. A total of 6.3 g of HCl was picked up by the mixture during the reaction. Product was worked up by dissolving in 50 ml water and 20 ml of acetonitrile and neutralizing to a pH of 8.4 with 11 g solid sodium carbonate. The 2-chloro-5-methylpyridine product was extracted into 3 portions each of 25 ml toluene and combined and thereafter washed 3 times with 15 ml portions water. The toluene solution was vacuum stripped at 50°C and 25 mm Hg. 1.2 g of 2-chloro-5-methylpyridine was recovered as a mobile liquid. It was identified by GLC and GC/MS.

Example 8

Preparation of 2-Hydroxy-5-methylpyridine

A 0.5 g portion of the product of Example 3 was mixed with 0.03 g p-toluene sulfonic acid and stirred for 2 hours while heat was applied by immersing in an oil bath at 110—130°C. Hydrogen chloride gas was given off.

This product was diluted with 50% aqueous ethanol, neutralized with sodium carbonate and analyzed by GLC. The yield of 2-hydroxy-5-methylpyridine was 0.25 g (67%) when compared with pure material. The product was isolated and characterized as follows: The neutralized solution of reaction product was vacuum stripped to dryness. The residue was treated with 2 mls ethanol and filtered. The filtrate was treated with activated carbon (Darco® G—60) heated to boiling and filtered. Vacuum stripping yielded 0.29 g. This material was characterized as predominantly 2-hydroxy-5-methylpyridine by GLC analysis. This product was then stirred with 0.5 ml acetone and recrystallized at room temperature and 0°C and filtered. The product was washed 3 times with 0.5 ml acetone. The washed product was 99.9% 2-hydroxy-5-methylpyridine, as determined by DSC analysis, with a melting point of 164°C. Analysis by MS matched an authentic sample.

Example 9

Preparation of 2-Chloro-5-methylpyridine

A solution containing 0.6 g of the product of Example 6 dissolved in dimethylformamide was treated with a slow stream of hydrogen chloride gas at 35—70°C until the exothermic reaction terminated. HCl gas addition was continued at 80°C for 1.5 hours, then at 100°C for 3 hours. Pick-up of HCl amounted to 1.8 g. The product was diluted with 6 parts of a 1:1 mixture of water and acetonitrile and neutralized to a pH 8.5 with sodium carbonate. This material was extracted with five 3 part portions of toluene. The toluene solution was analyzed by GLC and contained only 2-chloro-5-methylpyridine which indicated an essentially quantitative yield.

Example 10

Preparation of 2-Hydroxy-5-methylpyridine

A 0.5 g portion of the product of Example 6 was mixed with 0.03 gm p-toluenesulfonic acid and stirred under nitrogen at 130—135°C. GLC analysis indicated the product to be predominately 2-hydroxy-5-methylpyridine.

**Claims**

1. A compound having the general formula:

$$(X)(CN)CH\ CH_2CH(CH_3)CHO$$

wherein X is selected from Cl and Br.

2. 4-Formyl-2-pentenonitrile.

3. A process for preparing a compound of the formula

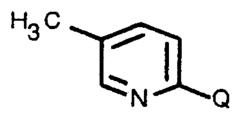

wherein Q is OH or X, and X is chloro or bromo characterized in that a compound of the formula

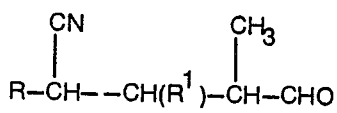

wherein R is X, $R^1$ is H and the broken line represents a single bond or R and $R^1$ are nothing and the broken line represents a double bond is subjected to treatment with a sulfonic acid to produce a compound wherein Q is OH or when R is X is subjected to acid catalysis or when R is nothing is reacted with HBr or HCl to produce a product wherein Q is X.

4. A process according to claim 3, for the production of a product when Q is OH characterized in that said sulfonic acid is p-toluene sulfonic acid.

5. A process according to claim 3, for the production of a product wherein Q is X characterized in that said compound of the formula

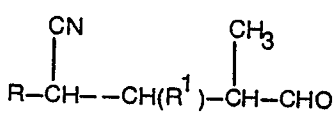

is one wherein R and $R^1$ are nothing and the broken line represents a double bond and said reaction with HCl or HBr is effected at a temperature gradually increasing from 35 to 100°C.

6. A process according to any one of preceding claims 3, 4 or 5, characterized in that said compound of the formula

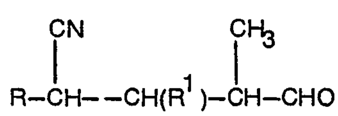

is prepared from a compound of the formula

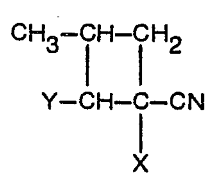

wherein X is chloro or bromo and Y is —$NR^1R^2$ wherein $R^1$ and $R^2$ are selected from individual substituted or unsubstituted alkyl groups having 1—6 carbon atoms and groups connected to form 5- or 6- membered

heterocyclic rings by either effecting mild hydrolysis to produce a compound wherein R is X, $R^1$ is H and the broken line represents a single bond or by reaction with aqueous acetic acid at 80—100°C to produce a product wherein R and $R^1$ are nothing and the broken line represents a double bond.

7. A process according to claim 6, characterized in that said compound of the formula

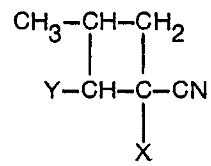

is prepared by reaction of a compound of the formula

$CH_3—CH_2—CHYY$ or $CH_3—CH=CHY$

with an acrylic compound of the formula $CH_2=C(X)CN$.

8. A process for preparing a compound useful in the process of claim 3, and of the formula

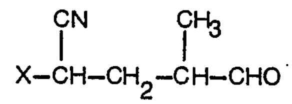

wherein X is chloro or bromo characterized in that a compound of the formula is prepared by mild hydrolysis of a compound of the formula

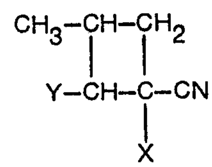

wherein X is chloro or bromo and Y is as defined in claim 6.

9. A process for preparing a compound useful in the process of claim 3, and of the formula

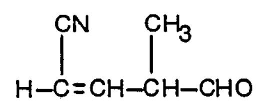

characterized in that a compound of the formula

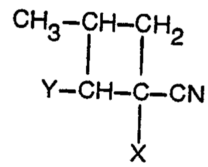

wherein X is chloro or bromo and Y is Morpholino is reacted with aqueous acetic acid at 80—100°C.

10. A process for preparing a compound useful in the process of either of claims 8 and 9, and of the formula

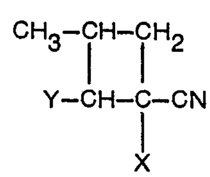

wherein X is chloro or bromo and Y is as defined in claim 1, characterized in that a compound of the formula

$CH_3—CH=CHY$ or $CH_3—CH_2—CHYY$

is reacted with an acrylic compound of the formula $H_2C=C(X)CN$.

**Patentansprüche**

1. Verbindung der allgemeinen Formel

$$(X)(CN)CH\ CH_2CH(CH_3)CHO$$

in der X aus Cl und Br ausgewählt ist.

2. 2-Formyl-2-pentenonotril

3. Verfahren zum Erzeugen einer Verbindung der Formel

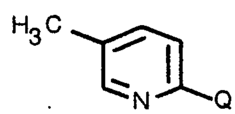

in der Q OH oder X ist und X Chloro oder Bromo ist, dadurch gekennzeichnet, daß eine Verbindung der Formel

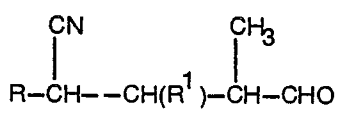

in der R X ist, $R^1$H ist und die gestrichelte Linie eine Einfachbindung darstellt oder R und $R^1$ nichts bedeuten und die gestrichelte Linie eine Doppelbindung darstellt, einer Behandlung mit einer Sulfonsäure unterworfen wird, so daß eine Verbindung erzeugt wird, in der Q OH ist oder, wenn R X ist, einer Säurekatalyse unterworfen wird, oder wenn R nichts bedeutet, mit HBr oder HCl umgesetzt wird, so daß ein Produkt erzeugt wird, in dem Q X ist.

4. Verfahren nach Anspruch 3 zum Erzeugen eines Produkts, in dem Q OH ist, dadurch gekennzeichnet, daß die Sulfonsäure die p-Toluolsulfonsäure ist.

5. Verfahren nach Anspruch 3 zum Erzeugen eines Produkts, in dem Q R ist, dadurch gekennzeichnet, daß bei einer Verbindung der Formel

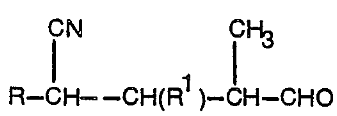

R und $R^1$ nichts bedeuten und die gestrichelte Linie eine Doppelbindung darstellt, die Umsetzung mit HCl oder HBr bei einer Temperatur bewirkt wird, die allmählich von 15 auf 100°C ansteigt.

6. Verfahren nach einem der vorhergehenden Ansprüche 3, 4 oder 5, dadurch gekennzeichnet, daß die Verbindung der Formel

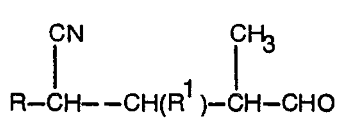

aus einer Verbindung der Formel

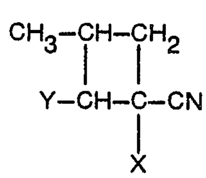

erzeugt wird, in der X Chloro oder Bromo ist, und

Y —$NR^1R^2$ ist, wobei $R^1$ und $R^2$ aus einzelnen substituierten oder nichtsubstituierten Alkylgruppen mit 1

bis 6 Kohlenstoffatomen und Gruppen ausgewählt ist, die zu 5- oder 6-gliedrigen heterozyklischen Ringen verbunden sind, indem entweder durch eine milde Hydrolyse eine Verbindung erzeugt wird, in der R X ist, $R^1$ H ist und die gestrichelte Linie eine Einfachbindung darstellt, oder durch eine Umsetzung mit wässriger Essigsäure bei 80 bis 100°C ein Produkt erzeugt wird, in dem R und $R^1$ nichts bedeuten und die gestrichelte Linie eine Doppelbindung darstellt.

7. Verfahren nach Anspruch 6, dadurch gekennzeichnet, daß zum Erzeugen der Verbindung der Formel

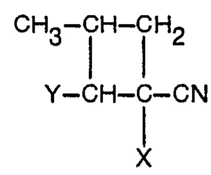

eine Verbindung der Formel

$CH_3—CH_2—CHYY$ oder $CH_3CH=CHY$

mit einer Acrylsäureverbindung der Formel $CH_2=C(X)CN$ umgesetzt wird.

8. Verfahren zum Erzeugen einer in dem Verfahren nach Anspruch 3 verwendbaren Verbindung der Formel

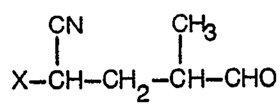

in der X Chloro oder Bromo ist, dadurch gekennzeichnet, daß eine Verbindung der Formel durch milde Hydrolyse einer Verbindung der Formel

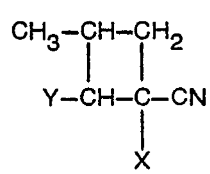

erzeugt wird, in der X Chlor oder Bromo ist und Y die im Anspruch 6 angegebene Bedeutung hat.

9. Verfahren zum Erzeugen einer im Verfahren nach Anspruch 3 verwendbaren Verbindung der Formel

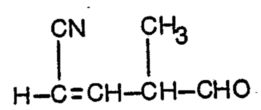

dadurch gekennzeichnet, daß eine Verbindung der Formel

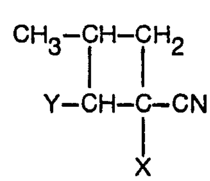

in der X Chlor oder Bromo ist und Y Morpholino ist, bei 80 bis 100°C mit wässriger Essigsäure umgesetzt wird.

10. Verfahren zum Erzeugen einer in dem Verfahren nach Anspruch 8 oder 9 verwendbaren Verbindung der Formel

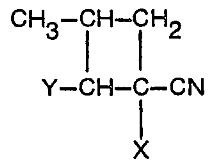

in der X Chloro oder Bromo ist und Y die im Anspruch 1 angegebene Bedeutung hat, dadurch gekennzeichnet, daß eine Verbindung der Formel

$$CH_3\text{—}CH\text{=}CHY \quad \text{oder} \quad CH_3\text{—}CH_2\text{—}CHYY$$

mit einer Acrylsäureverbindung der Formel $H_2C{=}C(X)CN$ umgesetzt wird.

**Revendications**

1. Composé répondant à la formule générale:

$$(X)(CN)CH\ CH_2CH(CH_3)CHO$$

dans laquelle X est choisi par Cl et Br.

2. 4-Formyl-2-pentenonitrile.

3. Procédé pour la préparation d'un composé de formule:

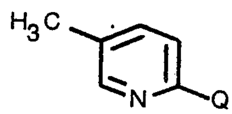

dans laquelle Q est OH ou X, et X est un atome de chlore ou de brome, caractérisé en ce qu'un composé de formule

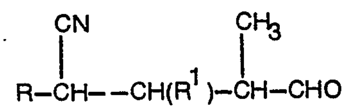

dans laquelle R est X, $R^1$ est H et la ligne en traits interrompus représente une liaison simple ou R et $R^1$ ne représentent rien et la ligne en traits interrompus représente une double liaison, est soumis à un traitement avec un acide sulfonique pour produire un composé dans lequel Q est OH, ou lorsque R est X, il est soumis à une catalyse acide, ou lorsque R n'est rien, il est mis en réaction avec HBr ou HCl pour produire un produit dans lequel Q est X.

4. Procédé selon la revendication 3, pour la production d'un produit dans lequel Q représente OH, caractérisé en ce que ledit acide sulfonique est l'acide p-toluènesulfonique.

5. Procédé selon la revendication 3, pour la production d'un produit dans lequel Q représente X, caractérisé en ce que ledit composé de formule

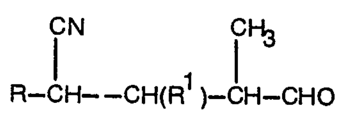

est un composé dans lequel R et $R^1$ ne représentent rien, et la ligne en traits interrompus représente un double liaison et ladite réaction avec HCl ou HBr est effectuée à une température s'élevant progressivement de 35 à 100°C.

6. Procédé selon l'une quelconque des revendications 3, 4 ou 5 précédentes, caractérisé en ce que l'on prépare ledit composé de formule

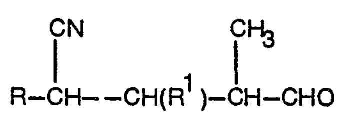

à partir d'un composé de formule

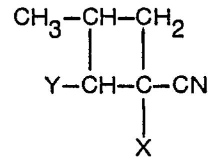

dans laquelle X est un atome de chlore ou de brome et

Y est —$NR^1R^2$ où $R^1$ et $R^2$ sont choisis parmi des groupes séparés, alkyle substitué ou non substitué ayant de 1 à 6 atomes de carbone et des groupes reliés pour former des noyaux hétérocycliques à cinq ou six chaînons, soit en effectuant une hydrolyse douce pour produire un composé dans lequel R est X, $R^1$ est H, et la ligne en traits interrompus représente un liaison simple, soit par réaction avec de l'acide acétique aqueux à 80—100°C pour produire un produit dans lequel R et $R^1$ ne représentent rien et la ligne en traits interrompus représente une double liaison.

7. Procédé selon la revendication 6, caractérisé en ce que l'on prépare ledit composé de formule

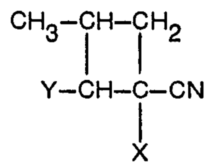

par réaction d'un composé de formule

$$CH_3—CH_2—CHYY \quad \text{ou} \quad CH_3CH=CHY$$

avec un composé acrylique de formule $CH_2=C(X)CN$.

8. Procédé pour la préparation d'un composé utile dans le procédé selon la revendication 3 et de formule

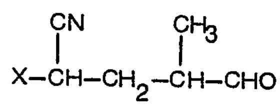

dans laquelle X est un atome de chlore ou de brome, caractérisé en ce que l'on prépare un composé ayant cette formule, par hydrolyse douce d'un composé de formule

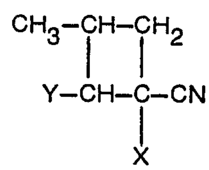

dans laquelle X est un atome de chlore ou de brome et Y est tel que défini dans la revendication 6.

9. Procédé pour la préparation d'un composé utile dans le procédé selon la revendication 3, et de formule

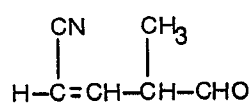

caractérisé en ce que l'on fait réagir un composé de formule

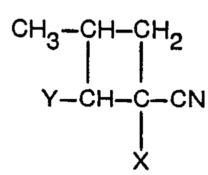

dans laquelle X représente un atome de chlore ou de brome et Y est un groupe morpholino, avec de l'acide acétique aqueux à 80—100°C.

10. Procédé pour la préparation d'un composé utile dans le procédé selon l'une quelconque des revendications 8 et 9, et de formule

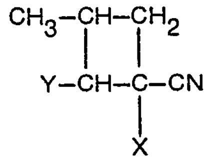

dans laquelle X est un atome de brome ou de chlore et Y est tel que défini dans la revendication 1, caractérisé en ce que l'on fait réagir un composé de formule

$$CH_3—CH=CHY \quad \text{ou} \quad CH_3—CH_2—CHYY$$

avec un composé acrylique de formule $H_2C=C(X)CN$.